# EUROPEAN PATENT APPLICATION

(11) **EP 3 095 386 A1**
(43) Date of publication of application: **23.11.2016**
(21) Application number: 14878885.4
(22) Date of filing: 05.12.2014
(51) Int. Cl.: A61B 8/00, A61B 8/13

(54) **PHOTOACOUSTIC MEASUREMENT APPARATUS AND SIGNAL-PROCESSING DEVICE AND SIGNAL-PROCESSING METHOD USED IN SAME**

(30) Priority: 16.01.2014 JP 2014005952; 18.11.2014 JP 2014233470
(71) Applicant: Fujifilm Corporation, Minato-ku Tokyo 106-8620 (JP)
(72) Inventor: IRISAWA, Kaku, Ashigarakami-gun Kanagawa 258-8538 (JP); HASHIMOTO, Atsushi, Ashigarakami-gun Kanagawa 258-8538 (JP)
(74) Representative: Klunker . Schmitt-Nilson . Hirsch
(86) International application number: PCT/JP2014/082287
(87) International publication number: WO 2015/107794

(57) **Abstract**

Disclosed are a photoacoustic measurement apparatus capable of improving ease of observation of a photoacoustic image even if an artifact is caused by a photoacoustic wave generated in a surface portion of a subject, on which measurement light is incident, and a signal processing device and a signal processing method for use therein. The photoacoustic measurement apparatus includes a probe having a light emission unit configured to emit measurement light to a subject and an acoustic wave detection unit provided in parallel with the light emission unit and configured to detect a photoacoustic wave generated in the subject by the emission of measurement light, an image generation unit which generates a photoacoustic image based on the photoacoustic wave detected by the acoustic wave detection unit, and a display processing unit which subjects the photoacoustic image to display processing for clarifying in the photoacoustic image an observation region from a position corresponding to a subject surface to an observable depth according to the interval between a reaching region on a contact plane of measurement light and the acoustic wave detection unit.

## Description

### BACKGROUND OF THE INVENTION

### 1. Field of the Invention

The present invention relates to a photoacoustic measurement apparatus which generates a photoacoustic image based on a photoacoustic signal generated in a subject, and a signal processing device and a signal processing method for use therein.

### 2. Description of the Related Art

In recent years, a noninvasive measurement method using a photoacoustic effect has been attracting attention. This measurement method irradiates a subject with pulse light having a predetermined wavelength (for example, the wavelength range of visible light, near-infrared light, or middle infrared light), detects an ultrasonic wave (photoacoustic wave) which is an elastic wave generated when an absorbing material in the subject absorbs the energy of pulse light, and quantitatively measures the concentration of the absorbing material. Examples of the absorbing material in the subject include glucose or hemoglobin in the blood. Furthermore, a technique which detects the photoacoustic wave and generates a photoacoustic image based on the detection signal is referred to as photoacoustic imaging (PAI) or photoacoustic tomography (PAT).

In conventional photoacoustic imaging, various display control methods or image processing methods for assisting a viewer to understand an image have been suggested.

For example, JP2013-005871A discloses a display control method which determines image quality at each point and displays a boundary for identifying regions with different image qualities.

JP2013-188461A discloses a photoacoustic imaging method which eliminates image information near a surface in a case of viewing a portion at depth.

JP2012-239784A discloses an image display method which synthesizes and displays an ultrasound image and a photoacoustic image, and displays a line for identifying a region of the photoacoustic image.

### SUMMARY OF THE INVENTION

On the other hand, the inventors have found that, in a case where a probe provided with a light emission unit and an acoustic wave detection unit in parallel to each other is used, a photoacoustic wave which is generated in a surface portion of a subject, on which measurement light is incident, propagates on the subject surface and is detected by the acoustic wave detection unit together with other signals, and causes an artifact (virtual image). The presence of an artifact may become an obstacle when observing a photoacoustic signal from a measurement target. However, the above-described photoacoustic wave which propagates on the subject surface is inevitably generated, and it is difficult to perform a photoacoustic measurement such that an artifact is completely not generated.

The invention has been accomplished in consideration of the above-described problem, and an object of the invention is to provide a photoacoustic measurement apparatus capable of improving ease of observation of a photoacoustic image in photoacoustic measurement even if an artifact is caused by a photoacoustic wave generated in a surface portion of a subject, on which measurement light is incident, and a signal processing device and a signal processing method for use therein.

In order to solve the above-described problem, a photoacoustic measurement apparatus of the invention comprises a probe having a light emission unit configured to emit measurement light to a subject and an acoustic wave detection unit provided in parallel with the light emission unit and configured to detect a photoacoustic wave generated in the subject by the emission of the measurement light, an image generation unit which generates a photoacoustic image based on the photoacoustic wave detected by the acoustic wave detection unit, and a display processing unit which subjects the photoacoustic image to display processing for clarifying in the photoacoustic image an observation region from a position corresponding to a subject surface to an observable depth according to the interval between a reaching region on a contact plane of the measurement light and the acoustic wave detection unit.

In the invention, the term "contact plane" means a plane which passes through the tip of the probe (that is, an intersection point of a probe surface in contact with the subject and a central axis of the acoustic wave detection unit) and is parallel to a detection surface of the acoustic wave detection unit. The term "reaching region" means a region where the contact plane and the measurement light intersect each other.

In the photoacoustic measurement apparatus of the invention, it is preferable that the display processing unit calculates the observable depth based on a calculation expression for calculating the observable depth from the interval or a correspondence table in which the interval and the observable depth are made to correspond to each other.

In the photoacoustic measurement apparatus of the invention, it is preferable that the probe is detachably and attachably mounted in an apparatus body having the display processing unit and has a storage unit which stores information for specifying the observable depth, and the display processing unit calculates the observable depth based on information acquired from the storage unit at the time of mounting the probe in the apparatus body.

Alternatively, in the photoacoustic measurement apparatus of the invention, it is preferable that the probe is detachably and attachably mounted in an apparatus body having the display processing unit and has a storage unit which stores the value of the observable depth according to the interval specific to the probe, and the display processing unit performs the display processing using the value of the observable depth acquired from the storage unit at the time of mounting the probe in the apparatus body.

In the photoacoustic measurement apparatus of the invention, it is preferable that the display processing unit corrects the observable depth based on at least one of a speed of sound in a measurement region of the subject, the intensity of the measurement light, the pulse width of the measurement light, or the presence or absence of an acoustic member on a detection surface of the acoustic wave detection unit.

In the photoacoustic measurement apparatus of the invention, it is preferable that the display processing is a process for displaying the observable depth by a line or processing for enhancing and displaying the observation region.

Alternatively, in the photoacoustic measurement apparatus of the invention, it is preferable that the display processing is a process for making an artifact region deeper than the observable depth in the photoacoustic image non-displayed. In this case, it is preferable that the display processing unit enables display of a partial region adjacent to the observation region in the non-displayed artifact region by the designation of an operator of the apparatus.

A signal processing device of the invention comprises a mounting portion in which a probe having a light emission unit configured to emit measurement light to a subject and an acoustic wave detection unit provided in parallel with the light emission unit and configured to detect a photoacoustic wave generated in the subject by the emission of the measurement light is detachably and attachably mounted, an image generation unit which generates a photoacoustic image based on the photoacoustic wave detected by the acoustic wave detection unit, and a display processing unit which subjects the photoacoustic image to display processing for clarifying in the photoacoustic image an observation region from a position corresponding to a subject surface to an observable depth according to the interval between a reaching region on a contact plane of the measurement light and the acoustic wave detection unit.

In the signal processing device of the invention, it is preferable that the display processing unit calculates the observable depth based on a calculation expression for calculating the observable depth from the interval or a correspondence table in which the interval and the observable depth are made to correspond to each other.

In the signal processing device of the invention, it is preferable that the display processing unit acquires information for specifying the observable depth stored in advance in the probe, which is detachably and attachably mounted in the signal processing device, at the time of mounting the probe in the signal processing device and calculates the observable depth based on this information.

Alternatively, in the signal processing device of the invention, it is preferable that the display processing unit acquires the value of the observable depth stored in advance in the probe, which is detachably and attachably mounted in the signal processing device, at the time of mounting the probe in the signal processing device and performs the display processing using the value.

In the signal processing device of the invention, it is preferable that the display processing unit corrects the observable depth based on at least one of a speed of sound in a measurement region of the subject, the intensity of the measurement light, the pulse width of the measurement light, or the presence or absence of an acoustic member on a detection surface of the acoustic wave detection unit.

In the signal processing device of the invention, it is preferable that the display processing is a process for displaying the observable depth by a line or for enhancing and displaying the observation region.

Alternatively, in the signal processing device of the invention, it is preferable that the display processing is a process for making an artifact region deeper than the observable depth in the photoacoustic image non-displayed. In this case, it is preferable that the display processing unit enables display of a partial region adjacent to the observation region in the non-displayed artifact region by the designation of an operator of the apparatus.

A signal processing method of the invention comprises generating a photoacoustic image based on a photoacoustic wave detected by a probe having a light emission unit configured to emit measurement light to a subject and an acoustic wave detection unit provided in parallel with the light emission unit and configured to detect the photoacoustic wave generated in the subject by the emission of the measurement light, and subjecting the photoacoustic image to display processing for clarifying in the photoacoustic image an observation region from a position corresponding to a subject surface to an observable depth according to the interval between a reaching region on a contact plane of the measurement light and the acoustic wave detection unit.

According to the photoacoustic measurement apparatus of the invention and the signal processing device and the signal processing method for use therein, the boundary between the observation region and the artifact region in the photoacoustic image is determined based on the interval between the reaching region on the contact plane of the measurement light and the acoustic wave detection unit. Since the interval is correlated with the position of the boundary in the photoacoustic image, if the interval is known in advance, it is possible to specify the range of the observation region in the photoacoustic image, and to perform display processing for clarifying the observation region. As a result, in the photoacoustic measurement, even if an artifact is caused by the photoacoustic wave generated in the surface portion, on which the measurement light is incident, it is possible to improve ease of observation of the photoacoustic image.

### BRIEF DESCRIPTION OF THE DRAWINGS

Fig. 1 is a schematic view showing the configuration of a photoacoustic measurement apparatus of a first embodiment.
Figs. 2A and 2B are schematic views showing the configuration of a probe of the first embodiment, and specifically, Fig. 2A is a sectional view of the probe when viewed from the front, and Fig. 2B is a sectional view of the probe when viewed from the side.
Fig. 3 is a schematic view showing a manner in which a photoacoustic wave generated from a blood vessel and a photoacoustic wave generated in a subject surface, on which measurement light is incident, are detected.
Figs. 4A to 4D are diagrams showing a photoacoustic image in which an artifact region is caused by a photoacoustic wave generated in a subject surface. Figs. 4E and 4F are respectively schematic views showing the positional relationship between a light emission unit and a transducer array when the photoacoustic images of Figs. 4A and 4D are generated.
Fig. 5 is a graph showing the relationship between a light guide plate-transducer interval and an observable depth.
Figs. 6A and 6B are schematic views showing an example of derivation of the interval between a reaching region of measurement light and an acoustic wave detection unit in a case where an emission axis of measurement light is directed outward, and specifically, Fig. 6A is a diagram showing a case where nothing is mounted on a detection surface, and Fig. 6B is a diagram showing a case where an acoustic member is mounted on a detection surface.
Fig. 7 is a flowchart showing a process of a photoacoustic measurement in the first embodiment.
Fig. 8 is a diagram showing another example of display processing.
Figs. 9A and 9B are diagrams showing another example of display processing.
Fig. 10 is a schematic view showing the configuration of a photoacoustic measurement apparatus of a second embodiment.
Fig. 11 is a flowchart showing a process of a photoacoustic measurement in the second embodiment.
Fig. 12 is a schematic view showing the configuration of a photoacoustic measurement apparatus of a third embodiment.

### DESCRIPTION OF THE PREFERRED EMBODIMENTS

An embodiment of the invention will be described referring to the drawings, but the invention is not limited to the embodiment. For ease of visual recognition, the scale and the like of each of components in the drawings are made to be appropriately different from the actual scale and the like.

### First Embodiment

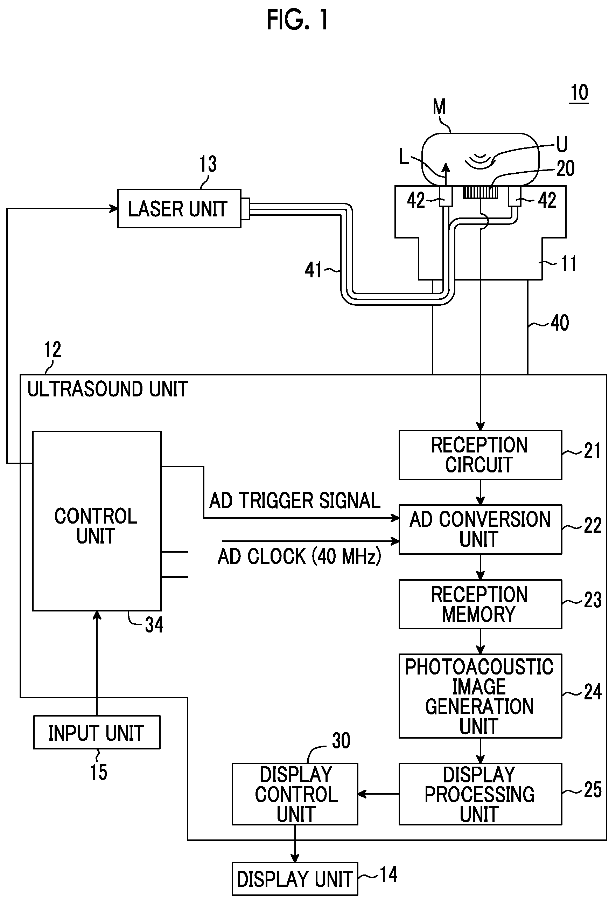

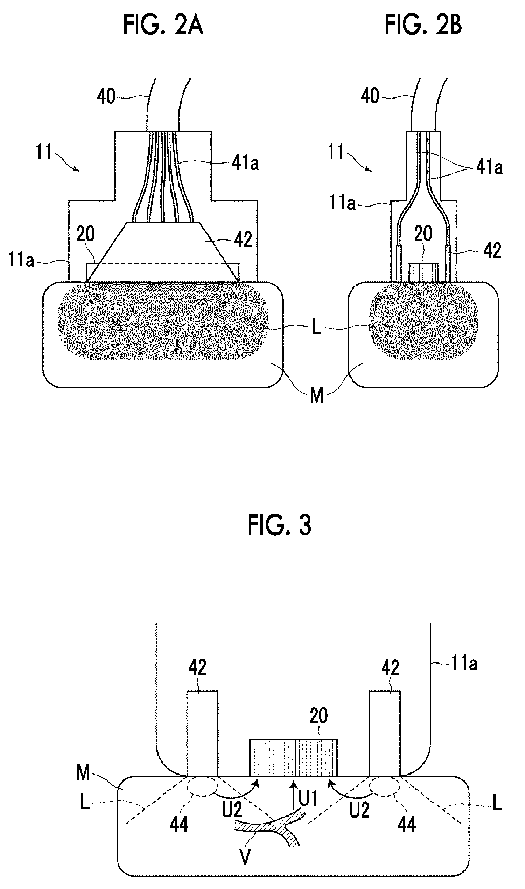

First, a photoacoustic measurement apparatus of first embodiment and a signal processing device and a signal processing method for use therein will be described. Fig. 1 is a schematic view showing the configuration of a photoacoustic measurement apparatus of this embodiment, and Figs. 2A and 2B are schematic views showing the configuration of a probe. Fig. 2A is a sectional view of the probe when viewed from the front, and Fig. 2B is a sectional view of the probe when viewed from the side.

A photoacoustic measurement apparatus 10 of this embodiment has a photoacoustic image generation function of, for example, generating a photoacoustic image based on a photoacoustic signal. Specifically, as shown in Fig. 1, the photoacoustic measurement apparatus 10 of this embodiment comprises a probe 11, an ultrasound unit 12, a laser unit 13, a display unit 14, and an input unit 15.

### Probe

The probe 11 irradiates a subject with an ultrasonic wave or detects an acoustic wave U propagating through the subject M. That is, the probe 11 can perform irradiation (transmission) of an ultrasonic wave to the subject M and detection (reception) of a reflected ultrasonic wave (reflected acoustic wave) reflected from the subject M. The probe 11 can perform detection of a photoacoustic wave generated in the subject M when an absorbent in the subject M absorbs a laser beam. In this specification, the term "acoustic wave" has a meaning including an ultrasonic wave and a photoacoustic wave. The term "ultrasonic wave" means an elastic wave transmitted from the probe and a reflected wave of the elastic wave, and the term "photoacoustic wave" means an elastic wave generated in the subject M by a photoacoustic effect according to irradiation of measurement light. Examples of the absorbent include a blood vessel, a metal member, and the like. The probe 11 is connected to the ultrasound unit 12 by a cable 40.

For example, as shown in Figs. 1, 2A, and 2B, the probe 11 of this embodiment comprises a transducer array 20, a bundle fiber 41 with a plurality of optical fiber wires 41a bundled, two light emission units 42 disposed with the transducer array 20 sandwiched therebetween, and a housing 11a including the transducer array 20, the bundle fiber 41, and the two light emission units 42.

The transducer array 20 has a plurality of ultrasound transducers (or acoustic wave detection elements) arranged in a one-dimensional or two-dimensional manner. In this embodiment, the transducer array 20 or each ultrasound transducer corresponds to an acoustic wave detection unit of the invention. Each ultrasound transducer is a piezoelectric element which is made of, for example, a polymer film, such as piezoelectric ceramics or polyvinylidene fluoride (PVDF). Each ultrasound transducer has a function of converting a reception signal to an electrical signal in a case where the acoustic wave U is received, and the electrical signal generated by the ultrasound transducer is output to a reception circuit 21 described below. The probe 11 is selected from a sector scanning type, a linear scanning type, and a convex scanning type according to an imaging region.

The bundle fiber 41 guides a laser beam from the laser unit 13 to the light emission units 42. The bundle fiber 41 is not particularly limited, and a known fiber, such as a quartz fiber, can be used. The bundle fiber 41 is branched into the optical fiber wires 41 a on an emission side and is connected to the light emission units 42.

The light emission units 42 are sections which irradiate the subject M with laser beams guided by the bundle fiber 41. As shown in Figs. 1 and 2B, in this embodiment, the two light emission units 42 are disposed on both sides of the transducer array 20 in an elevation direction (a direction perpendicular to the array direction of the transducer array and parallel to a detection surface) so as to face each other with the transducer array 20 sandwiched therebetween. As the light emission units 42, for example, a light guide plate can be used. The light guide plate is, for example, a plate which is obtained by performing special processing on the surface of an acrylic plate or a quartz plate and in which light incident on one end surface is uniformly emitted from the other end surface. In order to uniformly illuminate the subject surface on both sides of the transducer array 20, it is preferable that the width of the transducer array 20 in the array direction is nearly the same length as the width of the light guide plate. A diffusion plate may be provided at an incidence end or an emission end of the light guide plate.

### Laser Unit

The laser unit 13 has, for example, a solid-state laser source which emits a laser beam using a Q switch, and outputs a laser beam as measurement light L, with which the subject M is irradiated. For example, the laser unit 13 is configured to receive a trigger signal from a control unit 34 of the ultrasound unit 12 and to output a laser beam. It is preferable that the laser unit 13 outputs pulse light having a pulse width of 1 nsec to 100 nsec as a laser beam. For example, in this embodiment, a light source of the laser unit 13 is an alexandrite laser using a Q switch.

The wavelength of the laser beam is appropriately determined by the optical absorption characteristics of an absorbent in a subject to be a measurement target. For example, in a case where the measurement target is hemoglobin in a living body (that is, in a case of imaging a blood vessel), in general, it is preferable that the wavelength is a wavelength which belongs to a near-infrared wavelength range. The near-infrared wavelength range means a wavelength range of about 700 to 850 nm. However, the wavelength of the laser beam is not, of course, limited thereto. The laser beam may be a single wavelength, or may include a plurality of wavelengths (for example, 750 nm and 800 nm). In a case where the laser beam includes a plurality of wavelengths, the subject M may be simultaneously irradiated with the beams having these wavelengths or may be irradiated with the beams while the beams are alternately switched. The laser unit 13 may be a YAG-SHG-OPO laser or a Ti-Sapphire laser, which can output a laser beam in a near-infrared wavelength range, other than the alexandrite laser.

### Ultrasound Unit

The ultrasound unit 12 has a reception circuit 21, an AD conversion unit 22, a reception memory 23, a photoacoustic image generation unit 24, a display processing unit 25, a display control unit 30, and a control unit 34. The ultrasound unit 12 corresponds to a signal processing unit in the invention.

The control unit 34 controls the respective units of the photoacoustic measurement apparatus 10, and in this embodiment, comprises a trigger control circuit (not shown). The trigger control circuit sends a light trigger signal to the laser unit 13, for example, when activating the photoacoustic measurement apparatus. With this, in the laser unit 13, a flash lamp is turned on, and excitation of a laser rod is started. Then, the excitation state of the laser rod is maintained, and the laser unit 13 is placed in a state capable of outputting a pulse laser beam.

Thereafter, the control unit 34 transmits a Qsw trigger signal from the trigger control circuit to the laser unit 13. That is, the control unit 34 controls the output timing of the pulse laser beam from the laser unit 13 by the Qsw trigger signal. In this embodiment, the control unit 34 transmits a sampling trigger signal to the AD conversion unit 22 simultaneously with the transmission of the Qsw trigger signal. The sampling trigger signal becomes a sign of the start timing of sampling of a photoacoustic signal in the AD conversion unit 22. In this way, the sampling trigger signal is used, whereby it is possible to sample the photoacoustic signal in synchronization with the output of the laser beam.

The reception circuit 21 receives the photoacoustic signal detected by the probe 11. The photoacoustic signal received by the reception circuit 21 is transmitted to the AD conversion unit 22.

The AD conversion unit 22 samples the photoacoustic signal received by the reception circuit 21 to convert the photoacoustic signal to a digital signal. For example, the AD conversion unit 22 samples the photoacoustic signal in a predetermined sampling period based on an AD clock signal having a predetermined frequency input from the outside.

The reception memory 23 stores the photoacoustic signal sampled by the AD conversion unit 22. The reception memory 23 outputs data of the photoacoustic signal detected by the probe 11 to the photoacoustic image generation unit 24.

For example, the photoacoustic image generation unit 24 adds photoacoustic data stored in the reception memory 23 with a delay time according to the position of each ultrasound transducer to reconstruct data for one line, and generates data of a tomographic image (photoacoustic image) based on photoacoustic data of the respective lines. The photoacoustic image generation unit 24 may perform reconstruction using a circular back projection (CBP) method, instead of a delay addition method. The photoacoustic image generation unit 24 outputs data of the photoacoustic image generated as above to the display processing unit 25.

The display processing unit 25 subjects the photoacoustic image to display processing for clarifying an observation region (a region where a signal from a measurement target, such as a blood vessel, can be identified and which is primarily an observation target) on the upper side of the photoacoustic image. With this, the operator of the photoacoustic measurement apparatus easily observes an observation region even if an artifact region appears on the lower side of the photoacoustic image.

For example, Fig. 3 is a schematic view showing a manner in which a photoacoustic wave generated from a blood vessel V and a photoacoustic wave generated in a subject surface, on which measurement light is incident, are detected. When the subject M is irradiated with measurement light L, ideally, only a photoacoustic wave U1 from the blood vessel V has to be detected; however, actually, a photoacoustic wave U2 generated in a subject surface portion 44, on which measurement light L is incident, is also detected. The photoacoustic wave U2 causes an artifact (virtual image). The time from when the photoacoustic wave U2 is generated until the photoacoustic wave U2 is detected depends on the interval between each light emission unit 42 and the transducer array 20 (or each ultrasound transducer; the same applies to the following), and in more detail, the interval between a reaching region on a contact plane of measurement light L and the acoustic wave detection unit. That is, the larger the interval between each light emission unit 42 and the transducer array 20, the longer the distance at which the photoacoustic wave U2 propagates through the subject; thus, the time from when the photoacoustic wave U2 is generated until the photoacoustic wave U2 is detected is extended.

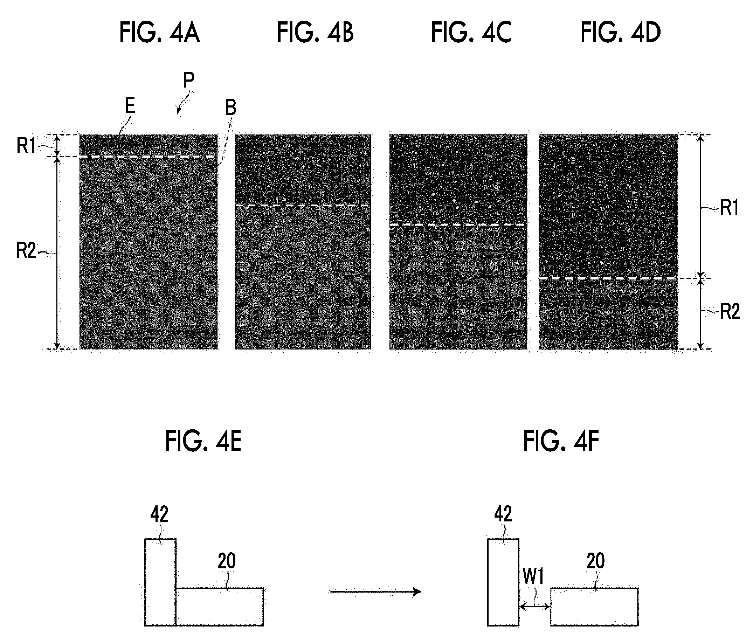

The time from when the photoacoustic wave U2 is generated until the photoacoustic wave U2 is detected affects the range in which an artifact region appears in the photoacoustic image. Figs. 4A to 4F are diagrams showing changes in size of an observation region R1 and an artifact region R2 depending on the interval W1 between the light emission unit 42 (light guide plate) and the transducer array 20. Specifically, Figs. 4A to 4D are diagrams showing a photoacoustic image P in which an artifact region R2 is caused by the photoacoustic wave U2 generated in the subject surface. Figs. 4E and 4F are respectively schematic views showing the positional relationship between the light emission unit 42 and the transducer array 20 when the photoacoustic images P of Figs. 4A and 4D are generated. From Figs. 4A to 4F, it is understood that the larger the interval W1 between the light emission unit 42 and the transducer array 20, the lower the boundary B between the observation region R1 and the artifact region R2. This is because the up-down direction of the photoacoustic image P corresponds to a time axis, and the larger the interval W1 between the light emission unit 42 and the transducer array 20, the later the time at which the signal of the photoacoustic wave U2 enters. In this embodiment, for example, although an example where the boundary between both regions is a depth line in which the ratio of the number of channels, in which an artifact appears, to the total number of channels of image data becomes 50%, the numerical value can be appropriately changed.

Accordingly, in the invention, the boundary is defined as an observable depth, a region from an upper end E of the photoacoustic image P to the observable depth is defined as an observation region, a region deeper than the observable region is defined as an artifact region, and an observation region of higher importance is clarified when observing the photoacoustic image, thereby improving ease of observation of the photoacoustic image.

A procedure for clarifying an observation region is, for example, as follows. First, the display processing unit 25 acquires the value of the interval W1 between the light emission unit 42 and the transducer array 20 and calculates the observable depth (for example, a length on the photoacoustic image or the number of pixels corresponding to the length) according to the value of the interval W1. Thereafter, the display processing unit 25 performs predetermined display processing with a region from the upper end of the photoacoustic image generated by the photoacoustic image generation unit 24 to the observable depth as an observation region. In this embodiment, display processing for displaying the observable depth by a line is performed (Figs. 4A to 4D).

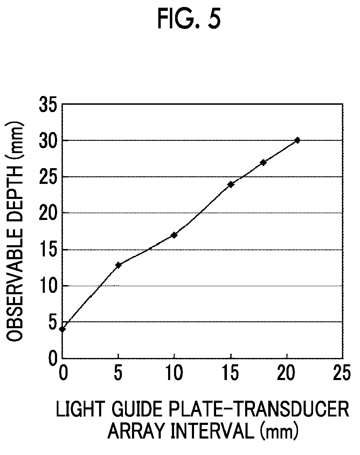

A method which acquires the value of the interval W1 may be a method which acquires the value of the interval W1 stored in advance in the photoacoustic measurement apparatus 10 or the probe 11, or may be a method which acquires the value of the interval W1 input by the operator of the apparatus with the input unit 15. As a method which acquires the observable depth from the value of the interval W1, for example, a method which calculates the observable depth based on a calculation expression for calculating the observable depth from the interval W1 can be used. As in this embodiment, in a case where the acoustic wave detection unit has a plurality of elements, as the interval W1, the interval between a representative element and the light emission unit 42 may be used, or the average value of the intervals between the respective elements and the light emission units 42 may be used. The inventors have found that the interval W1 and the observable depth have a predetermined correlation. Fig. 5 is a graph showing the relationship between the interval W1 between the light emission unit 42 and the transducer array 20 and the observable depth in a case where the positions of the light emission unit 42 and the transducer array 20 are changed as in Figs. 4E or 4F. For example, in this case, since it is recognized that the interval W1 and the observable depth have a substantially linear relationship, if a calculation expression obtained by approximation of the graph of Fig. 5 using a least-squares method is provided, it is possible to cope with any value input by the operator. As a method which calculates the observable depth from the value of the interval W1, a method which calculates the observable depth based on a correspondence table, in which the interval W1 and the observable depth are made to correspond to each other, may be used.

In Fig. 5, when the interval W1 is zero, this indicates a state where the light emission unit 42 and the transducer array 20 are in contact with each other (in other words, a state where a physical space between both of them is zero) as in Fig. 4E. However, in the invention, a way of defining "the interval between the light emission unit 42 and the transducer array 20" is not limited to the method described above. For example, in Figs. 4A to 4F and 5, a calculation expression may be derived by the above-described procedure with the distance between the optical axis of the light emission unit 42 and the central axis of the transducer array 20 as "the interval between the light emission unit 42 and the transducer array 20". This is because, in a case of numeralizing "the interval between the light emission unit 42 and the transducer array 20", for example, a portion which becomes a reference just affects an intercept of the graph of Fig. 5, and even if any reference is used, as a result, a calculation expression according to the used reference is derived. That is, for example, in a case of Figs. 4A to 4F and 5, even if the distance of the physical space between both of them or the distance between the optical axis and the central axis may be used as "the interval between the light emission unit 42 and the transducer array 20", "the interval between the reaching region on the contact plane of measurement light and the acoustic wave detection unit" is still considered when calculating the observable depth.

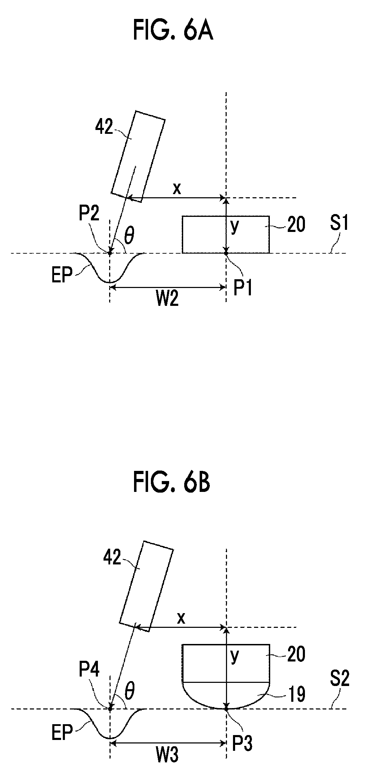

In a case where the optical axis is inclined with respect to the contact plane at the emission end of the light emission unit 42, "the interval between the reaching region on the contact plane of measurement light and the acoustic wave detection unit" can be defined as follows. For example, in a case where nothing is mounted on the detection surface as in Fig. 6A, an intersection point of the detection surface and the central axis of the transducer array 20 is referred to as P1, and a maximum point when an energy profile is approximated by a Gaussian distribution in the reaching region of measurement light L is referred to as P2. At this time, the point P1 becomes the tip of the probe through which the contact plane passes. Then, the distance W2 between the point P1 and the point P2 can be defined as "the interval between the reaching region and the acoustic wave detection unit". For example, as in Fig. 6B, in a case where an acoustic member 19, such as an acoustic lens, is mounted on the detection surface, an intersection point of a surface of the acoustic member 19 which comes into contact with the subject and the central axis of the transducer array 20 is referred to as P3, and a maximum point when an energy profile is approximated by a Gaussian distribution in the reaching region of measurement light L is referred to as P4. At this time, the point P3 becomes the tip of the probe through which the contact plane passes. Then, the distance W3 between the point P3 and the point P4 can be defined as "the interval between the reaching region and the acoustic wave detection unit".

It is preferable that the display processing unit 25 corrects the observable depth based on at least one of a speed of sound in a measurement region of the subject M, the intensity of measurement light, the pulse width of measurement light, or the presence or absence of an acoustic member on the detection surface of the acoustic wave detection unit. Basically, the observable depth is determined by the time from the photoacoustic wave U2 is generated until the photoacoustic wave U2 is detected. However, if the speed of sound in the measurement region of the subject M becomes slow, the distance at which the photoacoustic wave U2 propagates is constant, while the time at which the photoacoustic wave U2 is detected becomes slow; thus, an actual observable depth on the photoacoustic image becomes deeper than a primary observable depth determined in consideration of only the interval between the light emission unit 42 and the transducer array 20. If the intensity of measurement light L becomes large or the pulse width of measurement light L becomes short, background noise is not affected so much, while an artifact signal becomes large and the number of regions where an artifact signal is relatively predominant is increased; thus, the actual observable depth on the photoacoustic image becomes shallower than the primary observable depth. If an acoustic member, such as an acoustic lens, which is mounted on the detection surface of the acoustic wave detection unit, is placed on the detection surface, the actual observable depth on the photoacoustic image is deviated from the primary observable depth depending on acoustic impedance of the acoustic member. As a method which corrects the observable depth, a method which changes a coefficient in a calculation expression for calculating the observable depth according to the respective elements, such as the speed of speed or the pulse width, or a method which calculates the observable depth using a correspondence table including the respective elements as items can be used.

The display control unit 30 displays the photoacoustic image on the display unit 14, such as a display, based on photoacoustic image data subjected to predetermined display processing by the display processing unit 25. In a case where the probe 11 has the transducer array arranged in a two-dimensional manner and a plurality of photoacoustic images are acquired by probe scanning, for example, the display control unit 30 may create volume data based on these photoacoustic images and may display a composite image on the display unit 14 as a three-dimensional image.

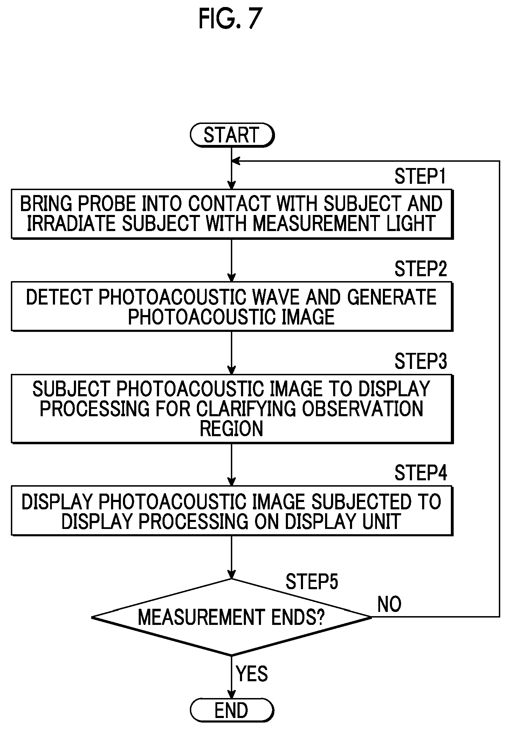

Hereinafter, a process of a photoacoustic measurement will be described referring to Fig. 7. Fig. 7 is a flowchart showing a procedure of a photoacoustic measurement in the first embodiment. First, the operator of the photoacoustic measurement apparatus 10 confirms the value of "the interval between the light emission unit and the transducer array" of the probe 11 determined as specifications, and the value is input to the apparatus 10. The operator holds the probe 11 and brings the tip of the probe 11 into contact with the subject. Thereafter, measurement light L is emitted from the light emission units 42, and the subject is irradiated with measurement light L (STEP1). A photoacoustic wave caused by irradiation of measurement light is detected by the probe 11, and a photoacoustic image is generated based on the signal (STEP2). The display processing unit 25 determines an observable depth based on the value of the interval input by the operator, and subjects the photoacoustic image to display processing for clarifying an observation region (STEP3). Thereafter, the photoacoustic image subjected to the display processing is sent to the display control unit 30 and is displayed on the display unit 14 (STEP4). Then, in a case of continuing a measurement, STEP1 to STEP4 are repeated; otherwise, the process ends (STEP5).

As above, the photoacoustic measurement apparatus of this embodiment and the signal processing device and the signal processing method for use therein calculate the boundary between the observation region and the artifact region in the photoacoustic image based on the interval between the reaching region on the contact plane of the measurement light and the acoustic wave detection unit. Since the interval is correlated with the position of the boundary in the photoacoustic image, if the interval is known in advance, it is possible to specify the range of the observation region in the photoacoustic image, and to perform the display processing for clarifying the observation region. As a result, in the photoacoustic measurement, even if an artifact is caused by the photoacoustic wave generated in the surface portion of the subject, on which the measurement light is incident, it is possible to improve ease of observation of the photoacoustic image.

### Design Change

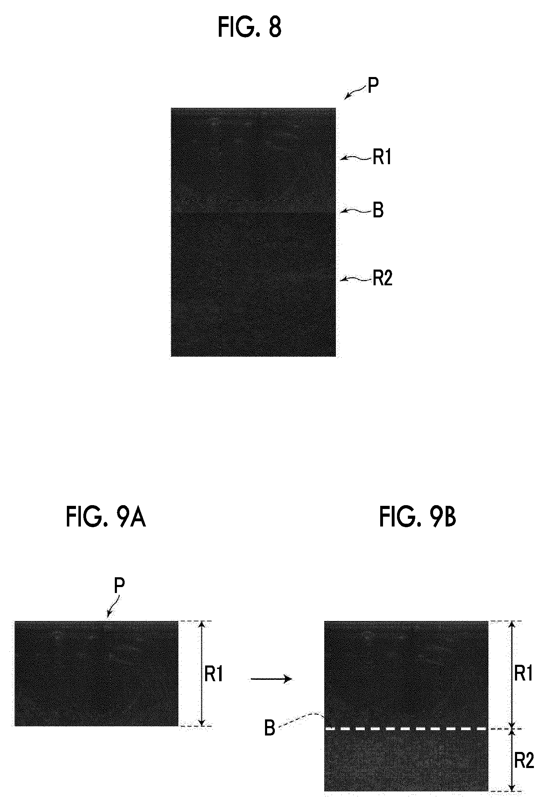

In the first embodiment described above, although the display processing for displaying the observable depth by a line has been described, the invention is not limited thereto. For example, as the display processing, a method which enhances an observation region compared to an artifact region by changing chromaticity, brightness, or saturation can be used. For example, in Fig. 8, an artifact region R2 is displayed in a color different from an observation region R1 and/or a color darker than the observation region R1 (for example, the observation region R1 is displayed in a color with red basic tone, and the artifact region R2 is displayed in a grayscale), whereby the observation region R1 is relatively enhanced.

As in Fig. 9A, for example, as the display processing, a method which deletes an artifact region R2 shown in Fig. 9B from a photoacoustic image P (that is, the artifact region R2 is not displayed as a display image) may be used. In this case, since a case where the operator desires to confirm a region near an observable depth including an artifact region is also considered, it is preferable to enable display of a partial region adjacent to the observation region R1 in the non-displayed artifact region R2 by the designation of the operator. For example, a form in which, if the operator drags a cursor to the lower end of the observation region R1, the non-displayed artifact region R2 is displayed by the dragged amount is considered.

In the first embodiment described above, although a case where the observable depth is calculated based on the interval between the light emission unit and the transducer array has been described, the invention is not limited thereto. For example, a form in which the operator inputs the value of the observable depth and the display processing unit 25 uses the value directly or corrects and uses the value may be made. Alternatively, information which is acquired by the display processing unit 25 is not particularly limited as long as information is information for specifying the observable depth, and for example, may be x (the distance between the position of the optical axis at the emission end of the light emission unit 42 and the central axis of the transducer array), y (the distance between the position of the optical axis at the emission end of the light emission unit 42 and the contact plane), and θ (the angle between the contact plane and the optical axis of measurement light) as shown in Figs. 6A and 6B. If x, y, and θ are obtained, the interval between the light emission unit and the transducer array is obtained, and the observable depth is obtained from the interval.

In the first embodiment described above, although a case where the display processing unit constantly performs the display processing has been described, the invention is not limited thereto. For example, the operator may select the on and off of the operation of the display processing unit 25 (that is, the necessity of for the display processing) using the input unit 15. In a case where an image is subjected to the display processing, an icon or the like representing that an image is subjected to the display processing so as to allow the operator to understand that an image is subjected to the display processing may be displayed on the display unit 14.

### Second Embodiment

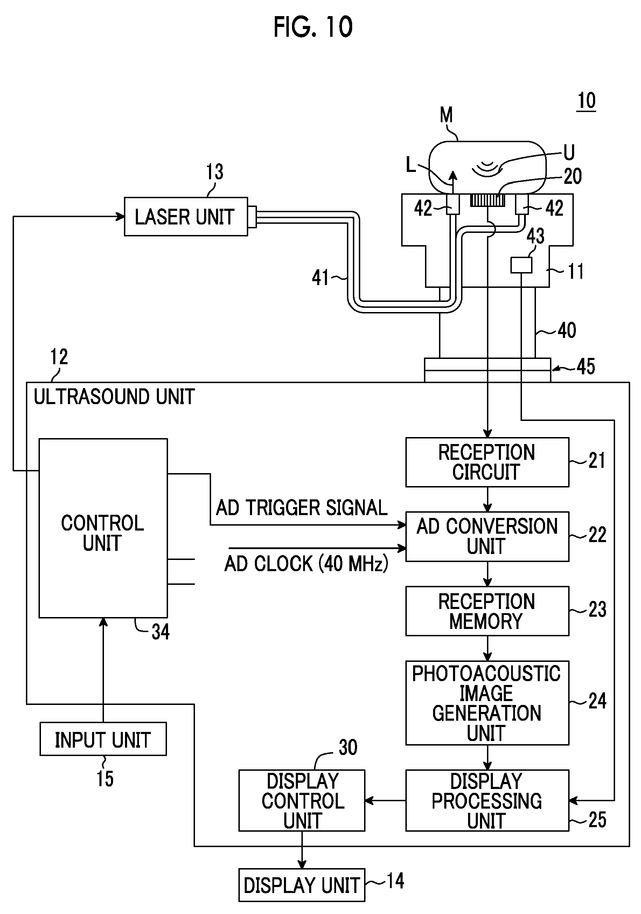

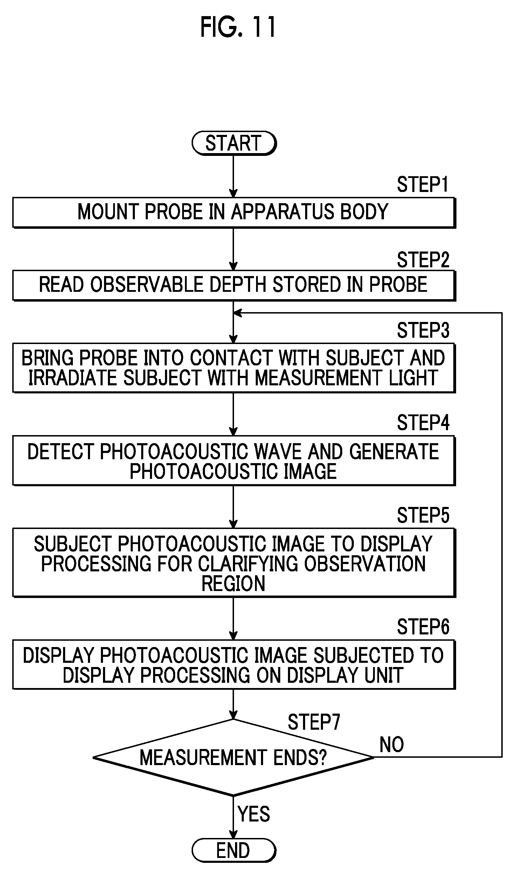

Next, a photoacoustic measurement apparatus of a second embodiment and a signal processing device and a signal processing method for use therein will be described. Fig. 10 is a schematic view showing the configuration of the photoacoustic measurement apparatus of this embodiment, and Fig. 11 is a flowchart showing a process of a photoacoustic measurement in the second embodiment. This embodiment is different from the first embodiment in that a probe 11 is detachably and attachably mounted in an apparatus body and the interval between the light emission unit and the transducer array is stored in advance in the probe 11. Therefore, detailed description of the same components as those in the first embodiment will not be repeated unless particularly necessary.

As shown in Fig. 10, a photoacoustic measurement apparatus 10 of this embodiment comprises a probe 11, an ultrasound unit 12, a laser unit 13, a display unit 14, and an input unit 15.

The probe 11 and the ultrasound unit 12 as the apparatus body are detachably and attachably provided through a connector 45. The probe 11 has a storage unit 43, and the storage unit 43 stores the value of the interval between each light emission unit 42 and the transducer array 20 specific to the probe 11 in advance.

The display processing unit 25 reads the value of the interval stored in the storage unit 43 when the probe 11 is mounted in the ultrasound unit 12 and calculates the observable depth based on this value.

Hereinafter, a process of a photoacoustic measurement will be described with reference to Fig. 11. First, for example, the operator of the photoacoustic measurement apparatus 10 selects a probe 11 for use in measurement from among a plurality of probes and mounts the probe 11 in the ultrasound unit 12 (STEP1). At this time, the storage unit 43 and the display processing unit 25 are connected to each other, and the display processing unit 25 reads the value of the interval from the storage unit 43 in the probe 11 (STEP2). Then, after the operator brings the tip of the probe into contact with the subject, measurement light L is emitted from the light emission units 42, and the subject is irradiated with measurement light L (STEP3). A photoacoustic wave caused by the irradiation of measurement light is detected by the probe 11, and a photoacoustic image is generated based on the signal (STEP4). The display processing unit 25 calculates an observable depth based on the read value of the interval and subjects the photoacoustic image to display processing for clarifying an observation region (STEP5). Thereafter, the photoacoustic image subjected to the display processing is sent to the display control unit 30 and is displayed on the display unit 14 (STEP6). Then, in a case of continuing a measurement, STEP3 to STEP6 are repeated; otherwise, the process ends (STEP7).

As above, the photoacoustic measurement apparatus of this embodiment and the signal processing device and the signal processing method for use therein calculate the boundary between the observation region and the artifact region in the photoacoustic image based on the interval between the reaching region on the contact plane of measurement light and the acoustic wave detection unit. Therefore, the same effects as in the first embodiment are obtained.

In this embodiment, the probe itself stores the value of the interval between each light emission unit 42 and the transducer array 20, and the value is automatically read into the display processing unit 25 in conjunction with mounting of the probe; thus, it is possible to save the time and effort of the operator for inputting the value. In a case where a plurality of probes are used properly according to purposes, it is possible to save the time and effort for changing the value for each probe. The same design change as described in the first embodiment is possible. The storage unit 43 may store the value of the observable depth according to the interval specific to the probe. In this case, the display processing unit 25 may read the observable depth from the storage unit 43 when the probe 11 is connected to the ultrasound unit 12 and may perform the display processing using the read value of the observable depth.

### Third Embodiment

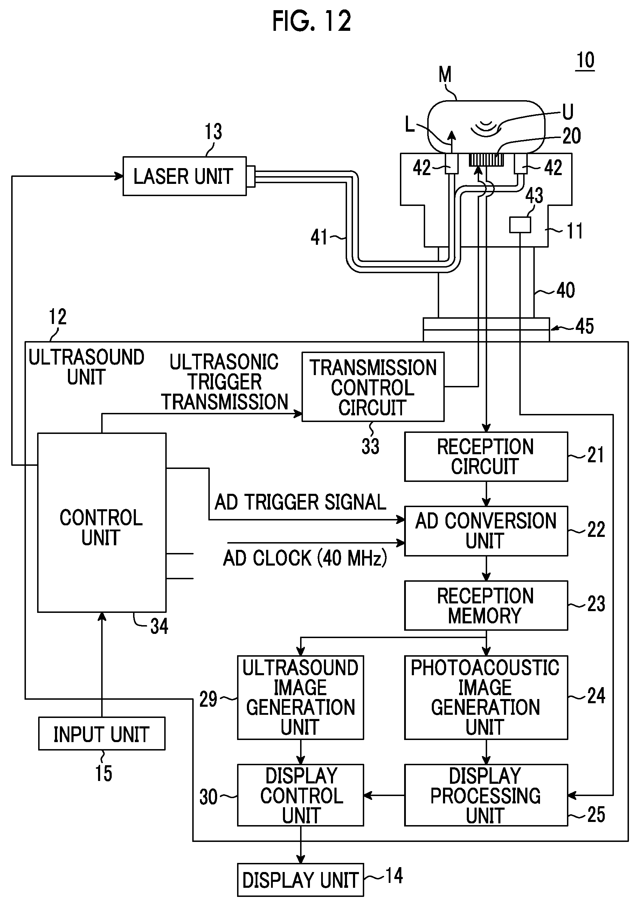

Next, a photoacoustic measurement apparatus of a third embodiment and a signal processing device and a signal processing method for use therein will be described. Fig. 12 is a schematic view showing the configuration of the photoacoustic measurement apparatus of this embodiment. This embodiment is different from the second embodiment in that an ultrasound image is generated in addition to a photoacoustic image. Therefore, detailed description of the same components as those in the second embodiment will not be repeated unless particularly necessary.

As shown in Fig. 12, a photoacoustic measurement apparatus 10 of this embodiment comprises a probe 11, an ultrasound unit 12, a laser unit 13, a display unit 14, and an input unit 15.

### Ultrasound Unit

The ultrasound unit 12 of this embodiment comprises an ultrasound image generation unit 29 and a transmission control circuit 33, in addition to the configuration of the photoacoustic measurement apparatus shown in Fig. 10.

In this embodiment, the probe 11 performs the output (transmission) of an ultrasonic wave to the subject and the detection (reception) of a reflected ultrasonic wave (reflected acoustic wave) of the transmitted ultrasonic wave from the subject, in addition to the detection of the photoacoustic signal. As an ultrasound transducer which performs transmission and reception of an ultrasonic wave, the transducer array 20 in the invention may be used, or a new ultrasound transducer which is provided in a different probe 11 for transmission and reception of an ultrasonic wave may be used. Transmission and reception of an ultrasonic wave may be separated. For example, transmission of an ultrasonic wave may be performed from a position different from the probe 11, and a reflected ultrasonic wave of the transmitted ultrasonic wave may be received by the probe 11.

The control unit 34 sends an ultrasonic transmission trigger signal to the effect of instructing the transmission control circuit 33 to perform ultrasonic transmission at the time of the generation of an ultrasound image. If the trigger signal is received, the transmission control circuit 33 transmits an ultrasonic wave from the probe 11. The probe 11 detects a reflected ultrasonic wave from the subject after transmitting the ultrasonic wave.

The reflected ultrasonic wave detected by the probe 11 is input to the AD conversion unit 22 through the reception circuit 21. The control unit 34 sends a sampling trigger signal to the AD conversion unit 22 according to the timing of ultrasonic transmission to start sampling of the reflected ultrasonic wave. The AD conversion unit 22 stores the sampling signal of the reflected ultrasonic wave in the reception memory 23. Either sampling of the photoacoustic signal or sampling of the reflected ultrasonic wave may be performed earlier.

The ultrasound image generation unit 29 performs signal processing, such as reconstruction processing, detection processing, and logarithmic conversion processing, based on (the sampling signal of) the reflected ultrasonic wave detected by the transducer array 20 of the probe 11 to generate data of an ultrasound image. For the generation of image data, similarly to the generation of image data in the photoacoustic image generation unit 24, a delay addition method or the like can be used. The ultrasound image generation unit 29 outputs data of the ultrasound image generated as above to the display control unit 30.

For example, the display control unit 30 displays the photoacoustic image and the ultrasound image individually or a composite image of the photoacoustic image and the ultrasound image on the display unit 14. For example, the display control unit 30 performs image composition by superimposing the photoacoustic image and the ultrasound image on each other.

In this embodiment, the photoacoustic measurement apparatus generates the ultrasound image in addition to the photoacoustic image. Therefore, in addition to the effects of the second embodiment, it is possible to observe a portion, which cannot be imaged in the photoacoustic image, with reference to the ultrasound image.

### Design Change

In the third embodiment, the same design change as described in the first embodiment is possible. In the third embodiment described above, although a case where the ultrasound image is not subjected to the display processing for clarifying the observation region has been described, the invention is not limited thereto. The boundary between the observation region and the artifact region in the photoacoustic image may be determined based on the interval between the reaching region on the contact plane of measurement light and the acoustic wave detection unit and the emission timing of measurement light, the range of the observation region in the photoacoustic image may be specified, and the ultrasound image of the same region as the observation region may be clarified.

### Explanation of References

- 10:: photoacoustic measurement apparatus
- 11:: probe
- 12:: ultrasound unit
- 13:: laser unit
- 14:: display unit
- 15:: input unit
- 9:: acoustic member
- 20:: transducer array
- 21:: reception circuit
- 22:: conversion unit
- 23:: reception memory
- 24:: photoacoustic image generation unit
- 25:: display processing unit
- 29:: ultrasound image generation unit
- 30:: display control unit
- 33:: transmission control circuit
- 34:: control unit
- 40:: cable
- 41:: bundle fiber
- 42:: light emission unit
- 43:: storage unit
- 45:: connector
- L:: measurement light
- M:: subject
- P:: photoacoustic image
- R1:: observation region
- R2:: artifact region
- U:: acoustic wave

## Claims

1. A photoacoustic measurement apparatus comprising:
a probe having a light emission unit configured to emit measurement light to a subject and an acoustic wave detection unit provided in parallel with the light emission unit and configured to detect a photoacoustic wave generated in the subject by the emission of the measurement light;
an image generation unit which generates a photoacoustic image based on the photoacoustic wave detected by the acoustic wave detection unit; and
a display processing unit which subjects the photoacoustic image to display processing for clarifying in the photoacoustic image an observation region from a position corresponding to a subject surface to an observable depth according to the interval between a reaching region on a contact plane of the measurement light and the acoustic wave detection unit.

2. The photoacoustic measurement apparatus according to claim 1,
wherein the display processing unit calculates the observable depth based on a calculation expression for calculating the observable depth from the interval or a correspondence table in which the interval and the observable depth are made to correspond to each other.

3. The photoacoustic measurement apparatus according to claim 1 or 2,
wherein the probe is detachably and attachably mounted in an apparatus body having the display processing unit and has a storage unit which stores information for specifying the observable depth, and
the display processing unit calculates the observable depth based on information acquired from the storage unit at the time of mounting the probe in the apparatus body.

4. The photoacoustic measurement apparatus according to claim 1,
wherein the probe is detachably and attachably mounted in an apparatus body having the display processing unit and has a storage unit which stores the value of the observable depth according to the interval specific to the probe, and
the display processing unit performs the display processing using the value of the observable depth acquired from the storage unit at the time of mounting the probe in the apparatus body.

5. The photoacoustic measurement apparatus according to any one of claims 1 to 4,
wherein the display processing unit corrects the observable depth based on at least one of a speed of sound in a measurement region of the subject, the intensity of the measurement light, the pulse width of the measurement light, or the presence or absence of an acoustic member on a detection surface of the acoustic wave detection unit.

6. The photoacoustic measurement apparatus according to any one of claims 1 to 5,
wherein the display processing is a process for displaying the observable depth by a line.

7. The photoacoustic measurement apparatus according to any one of claims 1 to 5,
wherein the display processing is a process for enhancing and displaying the observation region.

8. The photoacoustic measurement apparatus according to any one of claims 1 to 5,
wherein the display processing is a process for making an artifact region deeper than the observable depth in the photoacoustic image non-displayed.

9. The photoacoustic measurement apparatus according to claim 8,
wherein the display processing unit enables display of a partial region adjacent to the observation region in the non-displayed artifact region by the designation of an operator of the apparatus.

10. A signal processing device comprising:
a mounting portion in which a probe having a light emission unit configured to emit measurement light to a subject and an acoustic wave detection unit provided in parallel with the light emission unit and configured to detect a photoacoustic wave generated in the subject by the emission of the measurement light is detachably and attachably mounted;
an image generation unit which generates a photoacoustic image based on the photoacoustic wave detected by the acoustic wave detection unit; and
a display processing unit which subjects the photoacoustic image to display processing for clarifying in the photoacoustic image an observation region from a position corresponding to a subject surface to an observable depth according to the interval between a reaching region on a contact plane of the measurement light and the acoustic wave detection unit.

11. The signal processing device according to claim 10,
wherein the display processing unit calculates the observable depth based on a calculation expression for calculating the observable depth from the interval or a correspondence table in which the interval and the observable depth are made to correspond to each other.

12. The signal processing device according to claim 10 or 11,
wherein the display processing unit acquires information for specifying the observable depth stored in advance in the probe, which is detachably and attachably mounted in the signal processing device, at the time of mounting the probe in the signal processing device and calculates the observable depth based on this information.

13. The signal processing device according to claim 10,
wherein the display processing unit acquires the value of the observable depth stored in advance in the probe, which is detachably and attachably mounted in the signal processing device, at the time of mounting the probe in the signal processing device and performs the display processing using the value.

14. The signal processing device according to any one of claims 10 to 13,
wherein the display processing unit corrects the observable depth based on at least one of a speed of sound in a measurement region of the subject, the intensity of the measurement light, the pulse width of the measurement light, or the presence or absence of an acoustic member on a detection surface of the acoustic wave detection unit.

15. The signal processing device according to any one of claims 10 to 14,
wherein the display processing is a process for displaying the observable depth by a line.

16. The signal processing device according to any one of claims 10 to 14,
wherein the display processing is a process for enhancing and displaying the observation region.

17. The signal processing device according to any one of claims 10 to 14,
wherein the display processing is a process for making an artifact region deeper than the observable depth in the photoacoustic image non-displayed.

18. The signal processing device according to claim 17,
wherein the display processing unit enables display of a partial region adjacent to the observation region in the non-displayed artifact region by the designation of an operator of the apparatus.

19. A signal processing method comprising:
generating a photoacoustic image based on a photoacoustic wave detected by a probe having a light emission unit configured to emit measurement light to a subject and an acoustic wave detection unit provided in parallel with the light emission unit and configured to detect the photoacoustic wave generated in the subject by the emission of the measurement light; and
subjecting the photoacoustic image to display processing for clarifying in the photoacoustic image an observation region from a position corresponding to a subject surface to an observable depth according to the interval between a reaching region on a contact plane of the measurement light and the acoustic wave detection unit.
